# EUROPEAN PATENT SPECIFICATION

(11) **EP 3 873 281 B1**
(45) Date of publication and mention of the grant of the patent: **08.04.2026**
(21) Application number: 19787487.8
(22) Date of filing: 13.09.2019
(51) Int. Cl.: A24F 47/00, A24D 3/02, A24D 3/04, A24D 3/06, A61M 15/06

(54) **DISPOSABLE INHALATION DEVICE THAT RELEASES SMOKE WHICH IS NOT DIRECTLY INHALED**
EINWEGINHALATOR ZUR ABGABE VON NICHT DIREKT INHALIERTEM RAUCH
DISPOSITIF D'INHALATION JETABLE LIBÉRANT DE LA FUMÉE QUI N'EST PAS DIRECTEMENT INHALÉE

(30) Priority: 14.09.2018 MK 72618
(43) Date of publication of application: 08.09.2021
(73) Proprietor: TRPESKI, Sasho, 1000 Skopje (MK)
(72) Inventor: TRPESKI, Sasho, 1000 Skopje (MK)
(74) Representative: Danubia Patent & Law Office LLC
(86) International application number: PCT/MK2019/000002
(87) International publication number: WO 2020/055223

(56) References cited:
- WO-A1-2013/020280
- US-A1- 2004 261 807
- US-A1- 2009 277 465

## Description

### Technical field

"Disposable inhalation device that releases smoke which is not directly inhaled" is an invention which belongs to the area of devices which help people stop or reduce smoking as well as in the area of inhalers shaped like a cigarette for medical use and according to the International Patent Classification (IPC) includes the following markings: A61M 15/06, A24F 47/00 and A24F 47/002.

### Background art

Medical experts advise using inhalation for non-medical use in everyday life as a preventive way of preserving human health. Inhalation can also help to relax and improve the mental state of people inhaling. In addition, in medicine, inhalation is used for medical purposes as a method of treatment for certain diseases.

On the other hand, there is a large number of cigarette smokers who are trying to quit smoking, but only a small percentage succeed in it. One of the reasons why smokers cannot quit smoking is the habit of "doing something with their hands", and this has been confirmed by many scientific studies and surveys.

If we find a technical solution that will exploit the cigarette smokers' habit, in terms of their psychological need "to do something with their hands", which is also an addiction; and on the other hand, manage to exploit that habit for a useful purpose - that is inhalation, and at the same time help smokers reduce or stop smoking, in that case we have made great progress in terms of public health.

There are several types of inhalation devices with different technical solutions and different characteristics.

There is always room for improvement of the existing types of inhalation devices, that is, finding new technical solutions in order to have better features and performance than the existing ones.

Until now there is no invention or technical solution for an inhalation device for both medical and non-medical use possessing the following characteristics: simple and easy to use, mobile, small in size, efficient to use, capable of being manufactured in various dimensions as required, disposable, low production costs, flexible, requiring no electricity, no heating of the inhaling substance, no replenishment of the inhaled substance by changing the cartridges, no refilling of the inhalation fluid by dripping and releasing smoke which is not directly inhaled by the inhaler.

All of these parameters are the technical problem that has been posed and resolved by this invention, a problem whose final solution requires a patent protection.

The invention "Disposable inhalation device that releases smoke which is not directly inhaled" is a new type of inhalation device with new features and contains all the elements of the technical problem previously presented.

From state of technology point, there are many types of inhalation devices used to preventively protect people's health, as well as inhalers used in medicine. Different types of inhalation devices are characterized by different characteristics, different technical solutions and different ways of application.

Until now, there has been no invention or technical solution that has proved to be successful in combining the recommendation by medical experts for daily inhalation, as a preventive means for preserving human health, and at the same time contributing to the reduction or cessation of cigarette smoking, and which, in addition, can be used for medical purposes as needed. Furthermore, there is still no invention or a technical solution that will give the smoker a sense of authentic smoking and enjoyment of the smoking ritual and the same time its use will not impair his health. In this case we are considering the existing technical solutions of inhalation devices that do not use electricity for the inhalation process, do not use heating of the inhalation substance, inhalation devices that are small in size and can easily be held in hand. It is recognized that some of the existing technical solutions offer limited opportunities and few advantages.

As the background art of this invention we point out the following documents:
1. US 2004/261807 A1 (DUBE MICHAEL FRANCIS [US] ET AL) 30 December 2004 (2004-12-30);
2. US 2009/277465 A1 (KARLES GEORGIOS [US] ET AL) 12 November 2009 (2009-11-12);
3. WO 2013/020280 A1 (FEELLIFE BIOSCIENCE INTERNAT CO LTD [CN]; CHANG XINGHUI [CN] ET AL.) 14 February 2013 (2013-02-14).
4. EP 3 178 333 B1 (NINOMIYA, Yu, Tokyo 130-8603 (JP), • ITABASHI, Kenichi, Tokyo 130-8603 (JP)), 05.05.2021

These four documents contain the following background art:
Inhalation device that comprises a cylinder-shaped inhaler body formed by a sheath of thin, non-rigid material. that is, the body in the form of a cylinder may be deformed by the application of external pressure, where one end of the cylindrical body is filled with one type of material which absorb fluid and the material, that is, the material that absorb fluid are non-rigid and have the characteristic of allowing air to flow through them, in which material, a fragile canister is placed; the canister is filled with a volatile inhalation substance which does not need to be heated to evaporate or if it is otherwise manufactured, without a breakable canister, the material which absorb liquid, are moistened or soaked with drops of a volatile inhalation substance which does not need to be heated to evaporate, the end of the sheath, where the material is located, that is, the material that absorb fluid and allow air to flow through them, is perforated, so that in the inhalation process, the external atmospheric air enters through the perforated apertures inside the part where the material is located, that is, the materials that absorb fluid, and the other end of the cylindrical body is filled with combustible organic matter which, by combustion, is burned together with the sheath of the inhalation device in the part where the combustible organic matter is stored. The chip paper that wraps the filter has a vent hole through which ventilation air (outside air) is introduced into the filter for dilution of mainstream smoke.

All of these parameters are taken into consideration when finding a technical solution to this invention which requires a patent protection.

### Disclosure of the invention

The essence of this invention and the technical novelty of the product, in relation to the prior state of technology, is that the new inhalation device releases smoke which is created by the combustion of organic matter contained in the inhalation device and the smoke is not directly inhaled by the person who is inhaling. Smoke is not an integral part of the inhalation process, that is, there is another substance for inhalation present in the inhalation device.

Essential to this invention and the technical novelty of this product is that it is composed of three components: "inhalation section", "splitting section" and "organic matter combustion section", which together form a whole, yet all three parts function independently of one another. The technical novelty and particularly essential is that the "splitting section" of this invention located between the "inhalation section" and the "organic matter combustion section" does not allow air or smoke to move from the "inhalation section" to the "organic matter combustion section" and vice versa.

The most essential for this invention to work is that the "inhalation section", which is a cylinder, is physically blocked by a "splitting section" on one end, that is, on one side and there is no smoke penetration whatsoever from "the organic matter combustion section" into "the inhalation section", that is, this invention has no smoke main stream in conditions when the organic matter is burned in the "organic matter combustion section". Until now there hasn't been a state of the prior art such as an inhalation device that has "inhalation section" that is physically blocked on one side, so as not to cause smoke main stream in the "inhalation section" in conditions when the organic matter is burned in the "organic matter combustion section".
At the same time, in order for this invention to work, it is also essential that the "organic matter combustion section", which is a cylinder, is physically blocked by a "splitting section" on one end, that is one side, and from that side there is no smoke penetration whatsoever, from "the organic matter combustion part" into "the inhalation section", that is, this element: "the organic matter combustion section" does not produce smoke main stream under conditions when the organic matter is burned, that is, in this invention there is no passage of smoke from "the organic matter combustion section" into "the inhalation section".
Until now there hasn't been a state of the prior art such as an inhalation device that has a "organic matter combustion section" which is physically blocked on one side so as not to cause a smoke main stream in the inhalation device in conditions when the organic matter is burned.
"The splitting section" from this invention is the key element that do not allow smoke main stream in the inhalation device in conditions when the organic matter is burned.

Another important aspect of this invention is that in the process of inhalation, the external atmospheric air enters laterally, through the inhalation device's sheath, inside the body of the inhalation device, that is, it enters directly into the "inhalation section" through apertures perforated on the "inhalation section".

What is also important about this invention is that it is used for single use with no need for replenishment of the inhalation substance or replacing cartridges with an inhalation substance, or even refilling of the inhalation fluid by dripping.

Another essential aspect is that this inhalation device is made of materials that allow the sheath and the interior of the "inhalation section" not to be rigid, that is, to be flexible and in this manner, when we press down the inhalation section of the inhalation device, the sheath of the inhalation section deforms, and then by releasing the pressure, the place where the pressure was applied and the part where the body has been deformed returns to its original or approximately original position.

By pressing down on the body of inhalation device's "inhalation section", we also press the canister which is placed inside the material that fills the "inhalation section". The canister is filled with a volatile substance for inhalation, which does not need to be heated to evaporate. By pressing the canister, it breaks or ruptures and releases the volatile substance for inhalation and the inhalation substance is ready to be inhaled by the person doing the inhalation.

Apart from releasing the volatile substance from a canister, there is another way of performing this process in the same invention, not using a canister, but in the manufacture process of this inhalation device, the material contained within the "inhalation section" can be soaked with the volatile substance for inhalation, that is, moistened by drops of the volatile substance for inhalation and thus be ready for inhalation. The volatile substance for inhalation evaporates without being heated.

No matter which of these two methods is applied, after the inhalation, that is, when all volatile inhalation substance is expended, the inhalation device does not need to be refilled with a new inhalation substance by the person doing the inhalation, nor does the cartridge or the canister need to be replaced. As this inhalation device is disposable, once the entire inhalation substance is inhaled, the inhalation device is not used afterwards.

By burning the organic matter in the "organic matter combustion section" located opposite the "inhalation section", the "organic matter combustion section" burns together with the sheath of the "organic matter combustion section" and releases smoke which is not inhaled directly from the inhalation device by the person who is inhaling because of the "splitting section" that prevents smoke or air flow between the "inhalation section" and the "organic matter combustion section". The reason why this inhalation device is designed to emit smoke is because of the visual and psychological benefits that smoke provides, which is to alleviate stress and reduce the psychological distress of the person using the inhalation device.

This invention is named "Disposable inhalation device that releases smoke which is not directly inhaled" because although the device has an organic matter combustion section and there is a combustion of the organic matter in that section, this inhalation device does not cause smoke main stream, that is, the user of the inhalation device does not inhale smoke from the inhalation device whatsoever, due to the new features for which patent protection is required.

### Brief description of drawings

### Sheet No. 1/4 of FIG:

Sheet No. 1/4 of FIG illustrates four characteristic longitudinal sections of the invention "Disposable inhalation device that releases smoke which is not directly inhaled" together with components of the inhalation device.

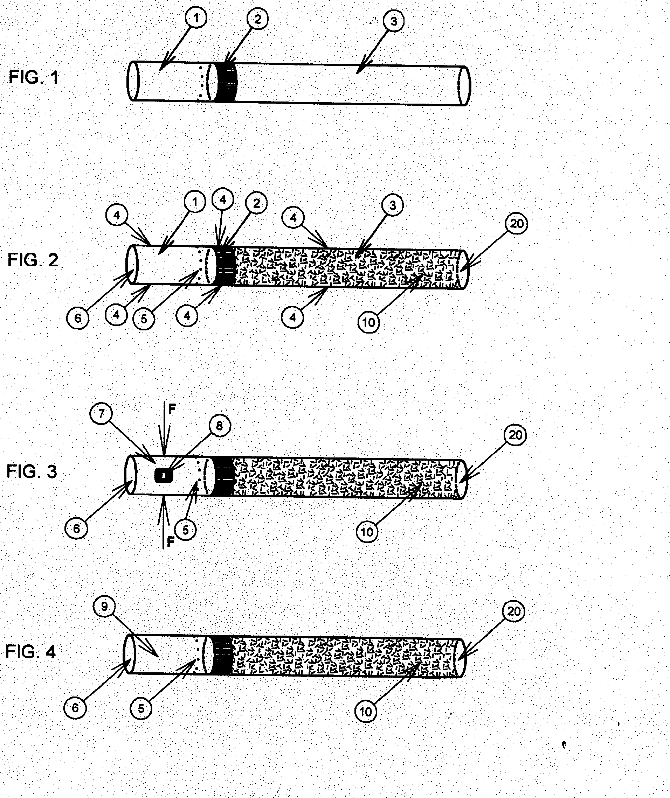

The first characteristic section (Fig. 1) shows the three parts of the inhalation device: the "inhalation section" (1), "the organic matter combustion section" (3) and the "splitting section" (2) located between "the inhalation section" "(1) and "section for combustion of organic matter "(3) and all these three parts together define the inhalation device.

The second characteristic section (Fig. 2) presents the sheath (4) which forms the body of the inhalation device, the perforated apertures in the inhalation section (5); the aperture (6) where the air flows out of the "inhalation section" (1), which air is mixed with the inhalation substance from inside the "inhalation section" (1) and it also shows the organic combustion matter (10) located inside the "organic matter combustion section" (3). This also shows the ending part (20) of the "organic matter combustion section"(3) which is ignited by a lighter, matches or other source of fire.

The third characteristic section (Fig. 3) shows the material (7) which fills the interior of the "inhalation section" (1), which has the characteristic of absorbing fluid and allowing air to flow through it and it also shows the canister (8) which contains a volatile substance for inhalation and is located in the cavity formed in the material inside the "inhalation section" (1). It also illustrates how to press down on the sheath "F" of the "inhalation section" (1) to break the canister (8).

The fourth characteristic section (Fig. 4) shows the second method of manufacturing the "inhalation section" (1) when no canister is fitted in the center and the material (9) or the materials that fill the interior of the "inhalation section" (1) in the center space do not form a small gap to accommodate the canister. In the second method of manufacturing, in the process of producing the inhalation device, the material(s) (9) which fill the interior of the "inhalation section" (1) are moistened with drops of the inhalation substance. The moistening is only in the process of manufacture and there is no additional moistening while using this inhalation device.

### Sheet No. 2/4 of FIG:

Sheet No. 1/4 of FIG shows two characteristic longitudinal sections of the invention "Disposable inhalation device that releases smoke which is not directly inhaled".

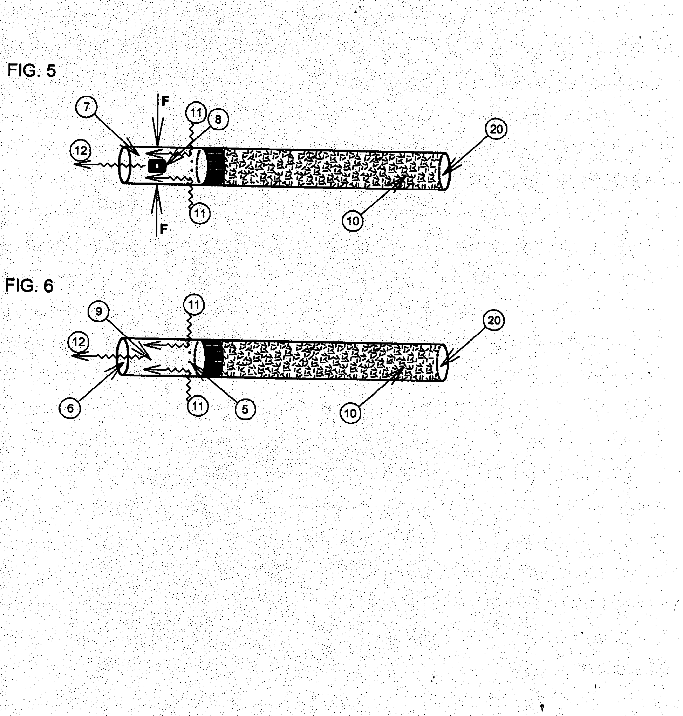

The first characteristic section (FIG. 5) shows the inflow (11) of external atmospheric air inside the "inhalation section" (1), which air mixes with the volatile inhalation substance that is released from the broken or ruptured canister (8) and it also shows the outflow (12) of the air together with the volatile inhalation substance outside the "inhalation section" (1).

The second characteristic section (FIG. 6) shows the inflow (11) of external atmospheric air inside the "inhalation section" (1), the air then mixes with the volatile inhalation substance with which the material (9), filling the interior o the "inhalation section", is moistened. It also shows the outflow (12) of the air together with the volatile inhalation substance outside the "inhalation section" (1).

### Sheet No. 3/4 of FIG:

Sheet No. 3/4 of FIG shows three characteristic longitudinal sections and characteristic cross-sections of the "splitting section" (2) according to the first method of manufacturing - with a seal.

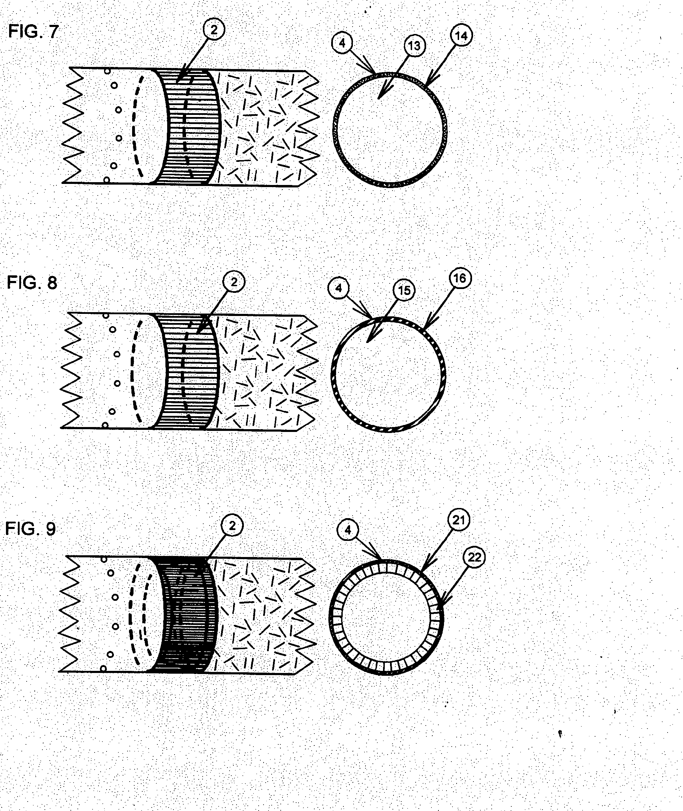

The first characteristic longitudinal and characteristic cross-sectional view (FIG. 7) shows the first method of manufacturing the "splitting section" (2) - with a seal (13) adhering (14) to the sheath (4).

The second characteristic longitudinal and characteristic cross-section (FIG. 8) shows the first method of manufacturing the "splitting section" (2) - with a seal (15) being fixed (16) to the sheath by means of other material.

The third characteristic longitudinal and characteristic cross-section (FIG. 9) shows the first method of manufacturing the "splitting section" (2) - with a seal (15) made of several different types of materials (21), (22). This Fig. shows only one of the several ways you can make a seal with many different types of materials.

### Sheet No. 4/4 of FIG:

Sheet No. 4/4 of FIG shows three characteristic longitudinal sections and characteristic cross-sections of the "splitting section" (2) according to the second and third methods of manufacturing: with a seal that is sealed and combined.

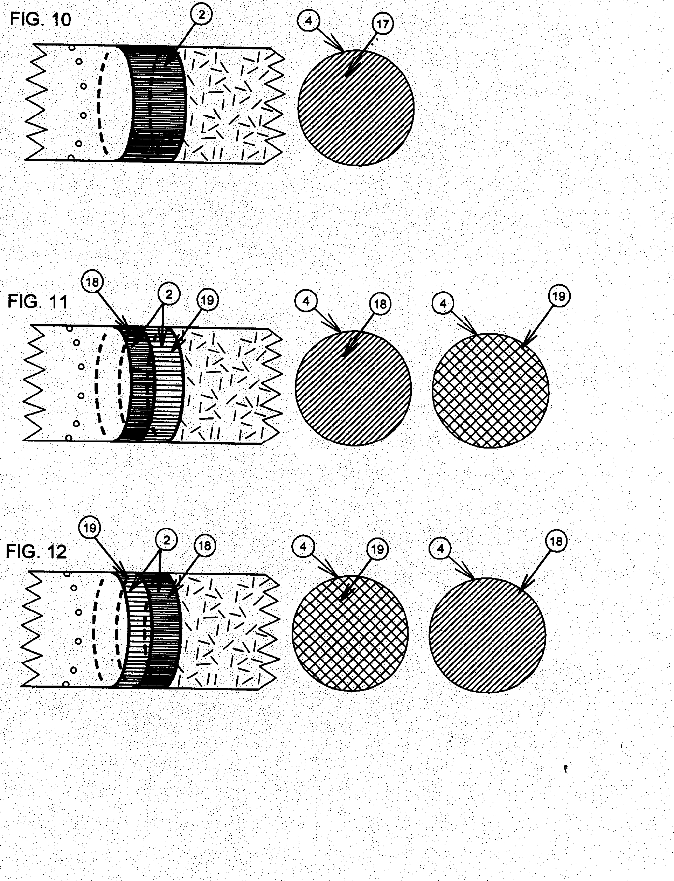

The first characteristic longitudinal and characteristic cross section (FIG. 10) shows the second method of manufacturing the "splitting section" (2) - a seal with sealing (17) which is attached to the sheath (4) after it has hardened.

The second characteristic longitudinal and characteristic cross-section (FIG. 11) shows the third method of manufacturing the "splitting section" (2) - combined (18) (19), part of the seal is made of solid material and part of it by sealing.

The third characteristic longitudinal and characteristic cross-section (FIG. 12) shows the third method of manufacturing the "splitting section" (2) - combined (18) (19) just like the previous one, part of the seal is made of solid material and the part of it by sealing only reversibly positioned.

### Explanation (a summary) of references:

(1) "Inhalation section"
(2) "Splitting section"
(3) "Organic matter combustion section"
(4) The sheath of the inhalation device
(5) Perforated apertures on the sheath of the "inhalation section" through which the external atmospheric air enters the interior of the inhalation section
(6) An opening through which the air from inside the "inhalation section", which is mixed with a volatile substance for inhalation, flows out from inside the inhalation device and enters the mouth of the person who is inhaling
(7) Material or materials which fill the interior of the "inhalation section" and which material or materials have the characteristic to absorb liquids
(8) Canister containing volatile substance for inhalation
(9) The second method of manufacturing the "inhalation section" is not to place a canister in the center, but only the material or materials having the characteristic of absorbing liquids and allowing air to flow through them
(10) Organic combustion material that fills the interior of the "organic matter combustion section"
(11) The inflow of outside atmospheric air inside the "inhalation section" at the time the person inhales
(12) The outflow of air outside the "inhalation section", where the air is mixed with a volatile inhalation substance, from inside the "inhalation section" at the time the person inhales
(13) A seal of solid material located in the "splitting section" that does not allow air or smoke to pass from the "inhalation section" to the "organic matter combustion section" and vice versa
(14) Glue that glues the seal (13) to the sheath of the splitting section and does not allow air or smoke to pass from the "inhalation section" to the "organic matter combustion section" and vice versa
(15) Seal of solid material located in the "splitting section" that does not allow air or smoke to pass from the "inhalation section" to the "organic matter combustion section" and vice versa
(16) Material that allows the seal (15) to be fixed to the sheath of the "splitting section" and does not allow air or smoke to move from the "inhalation section" to the "organic matter combustion section" and vice versa
(17) Seal located in the "splitting section" and made by sealing liquid or approximately liquid material, which hardens after sealing
(18) Part of a seal located in the "splitting section" made of solid material and combined with another part of seal (18) made by sealing liquid or approximately liquid material, which hardens after sealing
(19) The ending of "the organic matter combustion section" which is ignited by a lighter, matches or other source of fire
(20) An example of a combination of materials used to make the seal of the "splitting section"
(21) An example of a combination of materials used to make the seal of the "splitting section"

### Best Mode for Carrying the Inventions (detailed description of the method of realization of invention)

"Disposable inhalation device that releases smoke which is not directly inhaled" (hereinafter referred to as "inhaler") represents a whole which consists of three components: "inhalation section" (1), "organic matter combustion section"(3) and "splitting section" (2) which is located between the "inhalation section" (1) and "organic matter combustion section" (3).

The aforementioned three components, united together, form one whole which is the new invention: "A disposable inhalation device that releases smoke which is not directly inhaled".

The characteristic of this inhaler is that it is in the form of a cylinder.

The "splitting section" (2) is located between the other two parts and it separates and enables them, that is, "inhalation section" (1) and "organic matter combustion section" (3) to function independently of one another and in fact these two parts occupy both ends of the cylinder.

An inhaler can be manufactured in different dimensions, but in any case the dimensions must be appropriate so that one end of the inhaler can be inserted into the mouth at the moment of inhalation, and at the same time the inhaler can be smoothly held in one hand.

### INHALATION SECTION (1)

The "inhalation section" (1) is in the form of a cylinder. The cylinder of the "inhalation section" (1) at one end is bordered, that is, touched by the "splitting section" (2), and the other end of the cylinder is an opening used for drawing in air (6) by the person who is inhaling, that is, the person who inhales inserts this part of the cylinder into his mouth to inhale.

The cylinder of "the inhalation section" (1) has a sheath (4) made of a thin material that is not rigid, that is, when external pressure "F" is applied, or when you press down on the sheath, it does not break nor ruptures but it only deforms. The sheath is made of one type of material or several types of materials that may be glued together on one part of the sheath or the two materials may be positioned directly next to each other.

The material, that the "inhalation section" (1) is made of, must have the characteristic of preventing the volatile inhalation substance, which is located in the middle of "the inhalation section" (1), from leaking through the inhalation sheath or from evaporating through the inhalation sheath.

The material or the materials this sheath is made of are not protected by this application, that is, it can be made of already known materials with suitable characteristics which are previously described and materials that are a part of the "inhalation section" (1) of the inhaler.

The sheath of "the inhalation section" (1) is perforated (5) on one portion and the perforated apertures serve to let the external atmospheric air (11) into the "inhalation section" (1). The number of perforated apertures, their size, distance and the way they are positioned on "the inhalation section" (1) may vary.

Characteristic of the "inhalation section" (1) sheath is that the material from which this sheath is made does not end at the point where it borders the "splitting section" (2), but rather the material of the "inhalation section" (1) sheath proceeds and forms the sheath of the "splitting section" (2), or the cylinder of the "splitting section"(2) completely. Also the material of the "inhalation section"(1) sheath occupies a small part or occupies the whole and forms the sheath of "the organic matter combustion section" (3).

The sheath of "the inhalation section" can be externally designed.

The dimensions, or the length, of the "inhalation section" may vary with respect to the entire inhalation device.

The interior of the "inhalation section" (1), that is, the inside of the cylinder formed by the sheath of the "inhalation section" (1), is filled with material having the characteristic of absorbing fluid (9) and (7), and also not to be rigid. The interior of the "inhalation section" cylinder can be filled with one or two different types of materials that have the characteristic to absorb fluid and not be rigid. If the interior of the "inhalation section" is made of two different materials having the characteristic of absorbing fluid, they are positioned side by side in a longitudinal or transverse direction relative to the inside of the cylinder. The material, or materials, must have a characteristic to allow air (11) and (12), to flow through them in the inhalation process. The sheath and the interior of the "inhalation section" (1) are made of such materials that do not harm human health when the inhaler is inserted into the inhaler's mouth for inhalation.

Characteristic of the interior of "the inhalation section"(1) is that the material, or the materials, which fill the interior of "the inhalation section" (1) in the middle space form a small cavity in which the canister (8) is placed. The canister contains a volatile substance for inhalation that evaporates without preheating.

The canister may contain:
- volatile inhalation fluid or
- essential oil
- volatile gelatin
- aerosols
- inhalation gel
- a medicinal product for inhalation
- a combination of the above-given substances or
- another volatile substance for inhalation.

The above-given volatile inhalation substances are not protected by this application, that is, it may use familiar volatile substances for inhalation which do not need to be preheated to evaporate.

The dose and concentration of the volatile inhalation fluid, essential oil, gelatin, aerosols, inhalation gel or other volatile inhalation substance are determined by their manufacturer according to the characteristics and the need of the person who is inhaling.

The canister (8) may be partially or fully filled with a medicinal product for inhalation, whose properties, characteristics and dosage are given by the manufacturer of the medicinal product in question, and for which the manufacturer has obtained all the appropriate marketing authorizations. Accordingly, the section "Area of technology to which this invention pertains", in this application, clearly defines the inhaler as a device of medical use. "

The canister (8) is made of a material that does not allow the inhalation substance to leak or evaporate out of the canister until the canister (8) is pressed down (that is, external pressure is applied to the body of the "inhalation section" (1)). Once the "inhalation section" (1) is pressed externally, due to the characteristics and the flexibility of the material used for the sheath (4) and the interior of the "inhalation section" (9), (7), it deforms - at the same time, the canister (8) is pressed into the middle interior space and due to that pressure (8) the canister (8) is split or broken and the volatile substance leaks out of the canister, but inside the "inhalation section"(1). When the inhalation substance leaks out of the canister, it moistens the material (7), (9), that is, the two materials that are inside the "inhalation section" (1) and which have the characteristic to absorb fluid. In this way the volatile inhalation substance is ready for inhalation.

The material that the canister (8) is made of, its shape and size are not protected by this application, that is, familiar materials with suitable characteristics may be used. The material that the canister is made of does not harm human health.

There is another method of manufacturing the inhalation substance in the same invention:
In the second method, no canister is placed in the center of the "inhalation section" (1) and the material filling the interior of the "inhalation section" (1) does not form a small gap in the center space to set the canister (8).

In the second method, in the process of manufacturing the inhalation device, the material or materials that fill the interior of the "inhalation section" (1) are moistened with drops of the inhalation substance (9). The moistening is only in the process of manufacture and there is no further moistening while using this inhaler. Thus the "inhalation section" (1) is ready for use by the person who is inhaling.

Due to the method of primary packaging of the "Disposable inhalation device that releases smoke which is not directly inhaled", the primary packaging does not allow the inhalation substance to evaporate and is ready for inhalation by the inhaler (that is, the person inhaling) immediately after the opening of the primary packaging of this inhalation device.

Once the entire inhalation substance has been inhaled by the inhaler, this inhalation device is no longer used, that is, it is for single use only.

The airflow inside the "inhalation section" (1) is created after the inhaler has put the opening for inhaling (mouthpiece) in his/her mouth and after he/she has breathed in or inhaled (12). External atmospheric air enters (11) into the inner part of the "inhalation section" (1) through the perforated apertures (5), the air inside mixes with the inhalation substance, that the material which fills the inside of the "inhalation section" (1) is moistened with, so the mixed air together with the inhalation substance flows out of (12) the "inhalation section" (1) and flows into the mouth of the inhaler.

### SPLITING SECTION (2)

The "splitting section" (2) serves to separate the "inhalation section" (1) and the "organic matter combustion section" (3) so that the two separated sections function independently of each other, that is, it does not allow air or smoke to move from the "inhalation section" (1) to the "organic matter combustion section" (3) and vice versa. The "splitting section" (2) is located between the "inhalation section" (1) and the "organic matter combustion section" (3).

The "splitting section" (2) is in the form of a cylinder and has a sheath made of the same material, or materials, which form the sheath of the "inhalation section" (1). Practically speaking, the sheath forming the cylinder of the "inhalation section" (1) forms the cylinder of the "splitting section" (2) as well and is made of the same material, or materials, with no interruption in the sheath between the "inhalation section" (1) and "the splitting section"(2).

The dimensions, that is, the length of the "splitting section" may vary with respect to the entire inhalation device.

There are three different methods of manufacturing the interior of the "splitting section" (2) of this invention: with seal, seal with sealing and combined.

### First method of manufacturing the "splitting section" - with seal:

Inside the cavity formed by the sheath of the "splitting section" (1) is a seal (13), (15) with width, that is, a diameter equal or approximately equal to the diameter of the circular portion of the inner part of the cylinder sheath.

The seal (13), (15) is made of one type of material or a combination of several types of materials (21) (22). The seal (13), (15), (21), (22) which is inserted inside the sheath of the "splitting section" (1) is made of material or materials that have the characteristics of preventing the air from flowing through them, and thus preventing the air from flowing through the seal.

The material, that is, the materials that the seal (13), (15), (21) is made of, are not a novelty and are not protected by this application.
It is characteristic that the seal (13), (15) is glued (14) or fixed with another material (16) to the sheath of the "splitting section" (2) and thus it does not permit air or smoke flow between the "inhalation section" (1) and the "organic matter combustion section" (3).

It is characteristic that the seal made of several different types of materials (21), (22) is glued (14) or fixed with another material to the sheath of the "splitting section" (2) and thus it does not allow any air or smoke flow between the "inhalation section" (1) and the "organic matter combustion section" (3).

Part of the material, or part of the materials that the seal is made of, may penetrate the "inhalation section" (1) and / or the "organic matter combustion section" (3).

### Second method of manufacturing the "splitting section" - seal with sealing:

The cavity formed by the cylinder sheath of the "splitting section" (2) is sealed (17) with material in a liquid or approximately liquid state at the time of sealing. After a certain time, the sealing material (17) hardens.
The material that is sealed (17) in the inside of the "splitting section" (2) sheath is characterized by not allowing air to flow through the body of the material after it has hardened.

The material that the seal is made of is not a novelty and is not protected by this application.

It is characteristic that the material that is sealed in the interior of the "splitting section" (2) joins or adheres to the sheath (4) of the "splitting section" (2) and thus it does not allow any air or smoke flow between the "inhalation section" (1) and the "organic matter combustion section" (3).

### Third method of manufacturing the "splitting section" - combined:

Inside the cavity formed by the sheath of the "splitting section" (2) is a seal made with a combination of first and second method of manufacturing the splitting section.

One part of the seal is made of a material (19), that is, materials with a diameter equal or approximately equal to the diameter of the circular portion of the inner part of the cylinder sheath. The rest of the seal (18) is sealed with another material that is in liquid or approximately liquid state at the time of sealing, and then it hardens. This part of the seal (19) is made of one type of material or a combination of several types of material. The seal (19) placed inside the sheath of the "splitting section" (2) is made of material or materials that have the characteristics of preventing the flow of air through them, thereby preventing the flow of air through the seal (19).

The other part of the seal is sealed (18) with material in a liquid or approximately liquid state at the time of sealing. After a certain time, the material being sealed (18) hardens.

The material that is sealed (18) inside the sheath of the "splitting section" (2) is characterized by not allowing air to flow through the body of the material once it has hardened.

The material that is sealed (18) in the inside of the "splitting section" (2) joins or adheres to the sheath (4) of the "splitting section" (2) and thus it does not allow any air flow or smoke flow between the "inhalation section" (1) and the "organic matter combustion section" (3).

The seal is made of materials that are not a novelty and thus are not protected by this application.

### ORGANIC MATTER COMBUSTION SECTION (3)

The "organic matter combustion section" (3) is in the form of a cylinder. The cylinder of "organic matter combustion section" (3) at one end borders, that is, is touched by the "splitting section" (2), and the other end of the cylinder (20) of the "organic matter combustion section" (3) serves to ignite the organic matter that is inside the "organic matter combustion section" (3).

The cylinder of the "organic matter combustion section" (3) has a sheath (4) made of a thin material or several types of materials that gradually burn during ignition. The material, that is, the materials that this sheath is made of, are not protected by this application.

The characteristic of the material, which the "organic matter combustion section" (3) is made of, is that the sheath of this part may not end at the part where it borders the "splitting section" (2) but rather the material that the sheath is made of continues, that is, enters the "splitting section" (2).

The sheath of the "organic matter combustion section" can be externally designed. The interior of the "organic matter combustion section" (3), that is, the interior of the cylinder formed by the sheath of the "organic matter combustion section" (3) is filled with organic matter (10) which has the characteristic to burn and release smoke when it is ignited.

The dimensions, or the length of the "organic matter combustion section" (3) may vary with respect to the entire inhalation device.

### Possible manners of operation with disposable inhalation device that releases smoke which is not directly inhaled with examples

The person who is doing the inhalation takes the inhaler in his hand and with his/her fingers presses down "F" on the "inhalation section" (1) to break the canister (8) inside the inhalation section (1) and to release the volatile inhalation substance outside the canister (8), but still within the "inhalation section" (1).

If the inhaler is manufactured without the canister (8), as described above, then this procedure for pressing down on the "inhalation section" (1) of the inhaler's body is not performed.

The person doing the inhalation, uses a cigarette lighter, match or other source of fire, to light the end of the inhaler (20), that is, the end of the "organic matter combustion section" (3) and the organic matter (10) located inside the "organic matter combustion section" (3) starts burning together with the sheath of the "organic matter combustion section" (3) and it starts releasing smoke.

The person doing the inhalation puts the inhaler or the "inhalation section" (1) into his/her mouth and inhales (12). By inhaling, the external atmospheric air enters (11) into the interior of the "inhalation section" (1) through the perforated apertures (5) and mixes with the volatile substance for inhalation in the interior of the "inhalation section" (1). From there, the volatile inhalation substance together with the air in the "inhalation section" (1) flows out (12) of the "inhalation section" (1) and enters directly into the mouth of the person inhaling (12).

The smoke that is produced by the combustion of organic matter (10) in the "organic matter combustion section" (3) is because of the visual and psychological benefits that the smoke provides, which is to alleviate stress and reduce the psychological distress of the person using the inhaler.

## Claims

1. A disposable inhalation device that releases smoke which is not directly inhaled comprises a cylinder-shaped inhaler body formed by a sheath of thin, non-rigid material or several different types of thin, non-rigid materials, that is, the body in the form of a cylinder may be deformed by the application of external pressure, where one end of the cylindrical body is filled with one type of material or several different types of materials which absorb fluid and the material, that is, the materials that absorb fluid are non-rigid and have the characteristic of allowing air to flow through them, in which material, or materials, a fragile canister is placed; the canister is filled with a volatile inhalation substance which does not need to be heated to evaporate or if it is otherwise manufactured, without a breakable canister, the material, or the materials which absorb liquid, are moistened or soaked with drops of a volatile inhalation substance which does not need to be heated to evaporate, the end of the sheath, where the material is located, that is, the materials that absorb fluid and allow air to flow through them, is perforated, so that in the inhalation process, the external atmospheric air enters through the perforated apertures inside the part where the material is located, that is, the materials that absorb fluid, and the other end of the cylindrical body is filled with combustible organic matter which, by combustion, is burned together with the sheath of the inhalation device in the part where the combustible organic matter is stored, **characterized by** the inhalation device consists of three components: the inhalation section (1), the organic matter combustion section (3) and the splitting section (2), which components have the following characteristics: The inhalation section (1) is physically completely blocked for the smoke flow at one side by a splitting section (2) which makes it impossible for the smoke produced by the combustion of the organic matter in the organic matter combustion section (3) to enter the inhalation section (1) and thus this inhalation device does not have any smoke main stream, that is, when using the inhalation device the user does not inhale any smoke from the inhalation device due to the absence of smoke main stream, and in order for the inhalation section (1) to function, the external atmospheric air enters the inhalation section (1) only and only through perforated apertures in conditions when the organic matter in the organic matter combustion section (3) is burned; the splitting section (2) consists of a seal which can be adhered to the internal side of the splitting section's sheath or a seal which is fixed to the internal side of the splitting section's sheath, and the splitting section (2) physically completely blocks the smoke flow on one side of the inhalation section (1) so that the smoke, produced by the combustion of the organic matter in the organic matter combustion section (3) does not enter the inhalation section (1) and at the same time the splitting section (2) physically completely blocks the smoke flow on one side of the organic matter combustion section (3) in order to prevent the smoke main stream from appearing in the inhalation device, that is, the smoke remains in the organic matter combustion section (3) and the user does not inhale any smoke through the inhalation device at all; the organic matter combustion section (3) is physically completely blocked for smoke flow at one side by a splitting section (2) and therefore it is not possible for the smoke produced by combustion of the organic matter to enter into the inhalation section (1), that is, the inhalation device does not have any smoke main stream.

2. A disposable inhalation device that releases smoke which is not directly inhaled according to claim 1, **characterized by** the splitting section (2) is positioned between the inner physically blocked for the smoke flow side of the inhalation section (1) and the the inner physically blocked for the smoke flow side of organic matter combustion section (3).

3. A disposable inhalation device that releases smoke which is not directly inhaled according to the claims 1 and 2, **characterized by** the splitting section (2) consists of a seal which is located inside the sheath and is made of one type of material (13) having the feature of preventing the flow of air or smoke through the material, thus the constituting components of the inhalation device: the inhalation section (1) and the organic matter combustion section (3) are fully functionally separated by the seal which enables the prevention of air or smoke flow between the inhalation section (1) and the organic matter combustion section (3).

4. A disposable inhalation device that releases smoke that is not directly inhaled according to claims 1, 2, and 3, **characterized by** the seal is affixed to the internal side (14) of the sheath and does not allow air or smoke to flow into the part between the seal and the sheath, thus the inhalation section (1) and the organic matter combustion section (3) are completely functionally separated and there is no air or smoke flow between the inhalation section (1) and the organic matter combustion section (3).

5. A disposable inhalation device that releases smoke which is not directly inhaled according to claims 1 and 2, **characterized by** the splitting section (2) consists of a seal located inside the sheath and made of one type of material ( 15) which has the characteristic of not allowing air or smoke to flow through it, thus the constituting components of the inhalation device: the inhalation section (1) and the organic matter combustion section (3) are fully functionally separated by the seal and there is no air or smoke flow between the inhalation section (1) and the organic matter combustion section (3) and the seal is affixed to the internal side (16) of the sheath by another material and does not allow air or smoke to flow into the part between the seal and the sheath, thus the inhalation section (1) and the organic matter combustion section (3) are fully functionally separated and there is no air or smoke flow between the inhalation section (1) and the organic matter combustion section (3).

6. A disposable inhalation device that releases smoke which is not directly inhaled according to claims 1 and 2, **characterized by** the splitting part (2) consists of a seal located inside the sheath and made of two or several different types of material (21) and (22) which have the characteristic of not allowing air or smoke to flow through them, thus the constituting components of the inhalation device: the inhalation section (1) and the organic matter combustion section (3) are fully functionally separated by the seal and there is no air or smoke flow between the inhalation section (1) and the organic matter combustion section (3).

7. A disposable inhalation device that releases smoke which is not directly inhaled according to claims 1, 2 and 6, **characterized by** the seal is affixed to the sheath by means of another material or glued to the internal side of the sheath and does not allow air or smoke flow in the part between the seal and the sheath, thus the inhalation section (1) and the organic matter combustion section (3) are completely functionally separated and there is no air flow or smoke flow between the inhalation section (1) and the organic matter combustion section (3).

8. A disposable inhalation device that releases smoke that is not directly inhaled according to claims 1 and 2, **characterized by** the splitting part (2) consists of a seal located inside the sheath and made by sealing, that is, the cavity formed by the cylinder sheath of the splitting part (2) is sealed with a material which is in liquid or approximately liquid state at the time of the sealing and after a certain time, the sealing material hardens.

9. A disposable inhalation device that releases smoke that is not directly inhaled according to claims 1, 2 and 8, **characterized by** after the sealing process, once the material has hardened it has the characteristic of preventing air or smoke flow through it, thus the inhalation section (1) and the organic matter combustion section (3) are fully functionally separated by the seal and it does not allow air flow or smoke flow between the inhalation section (1) and the organic matter combustion section (3).

10. A disposable inhalation device that releases smoke which is not directly inhaled according to claims 1, 2, 8 and 9, **characterized by** after the material that the sheath is made of hardens, the material adheres to the sheath of the splitting section (2) and thus it does not allow any air flow or smoke flow in the part between the seal and the sheath, consequently the "inhalation section" (1) and the "organic matter combustion section" (3) are fully functionally separated and there is no possibility for air flow or smoke flow between the inhalation section (1) and the organic matter combustion section (3).

11. A disposable inhalation device that releases smoke that is not directly inhaled according to claim 1, and 2, **characterized by** the splitting part (2) is composed of a seal located inside the sheath and the seal is made of: a solid material and material (19) and by sealing (18).

12. A disposable inhalation device that releases smoke which is not directly inhaled according to claims 1, 2, and 11, **characterized by** the materials (18), (19) that the seal is made of, have the characteristic of preventing air or smoke flow through them and thus the inhalation section (1) and the organic matter combustion section (3) are fully functionally separated by the seal and there is no possibility for air flow or smoke flow between the inhalation section (1) and the organic matter combustion section (3).

13. A disposable inhalation device that releases smoke which is not directly inhaled according to claims 1, 2, 11 and 12, **characterized by** the seal is affixed to the sheath by means of another material or glued to the internal side of the sheath and does not allow air or smoke flow in the part between the seal and the sheath, thus the inhalation section (1) and the organic matter combustion section (3) are completely functionally separated and there is no possibility for air flow or smoke flow between the inhalation section (1) and the organic matter combustion section (3).

14. A disposable inhalation device that releases smoke which is not directly inhaled according to claim 1, 2, 3, 4, 5, 6, 7, 8, 9, 10, 11, 12 and 13, **characterized by** the material or the materials that the seal of the splitting part (2) is made of, partially or fully penetrate the inhalation section (1) and / or the organic matter combustion section (3).

## Patentansprüche

1. Ein Einweg-Inhalationsgerät, das Rauch freisetzt, der nicht direkt inhaliert wird, umfasst einen zylinderförmigen Inhalator-Körper, der aus einer Hülle aus dünnem, nicht starrem Material oder mehreren verschiedenen Arten von dünnem, nicht starrem Material gebildet ist. Das bedeutet, dass der Körper in Form eines Zylinders durch die Ausübung von äußerem Druck verformt werden kann, wobei ein Ende des zylindrischen Körpers mit einer Art von Material oder mehreren verschiedenen Arten von Materialien gefüllt ist, die Flüssigkeit absorbieren. Das Material bzw. die Materialien, die Flüssigkeit absorbieren, sind nicht starr und haben die Eigenschaft, Luft durchzulassen. In diesem Material oder diesen Materialien befindet sich ein zerbrechlicher Behälter, der mit einer flüchtigen Inhalationssubstanz gefüllt ist, die zum Verdampfen nicht erhitzt werden muss, oder, wenn er anders hergestellt ist, ohne zerbrechlichen Behälter, wird das Material oder werden die Materialien, die Flüssigkeit absorbieren, mit Tropfen einer flüchtigen Inhalationssubstanz befeuchtet oder getränkt, die zum Verdampfen nicht erhitzt werden muss. Das Ende der Hülle, an dem sich das Material bzw. die Materialien befinden, die Flüssigkeit absorbieren und Luft durchlassen, ist perforiert, sodass beim Inhalieren die Außenluft durch die perforierten Öffnungen in den Teil eindringt, in dem sich das Material befindet, d. h. die Materialien, die Flüssigkeit absorbieren. Das andere Ende des zylindrischen Körpers ist mit brennbarer organischer Substanz gefüllt, die bei der Verbrennung zusammen mit der Hülle der Inhalationsvorrichtung in dem Teil verbrannt wird, in dem die brennbare organische Substanz gespeichert ist. Das Inhalationsgerät besteht aus drei Komponenten: dem Inhalationsabschnitt (1), dem Abschnitt zur Verbrennung der organischen Substanz (3) und dem Spaltabschnitt (2). Die Komponenten weisen die folgenden Merkmale auf: Der Inhalationsabschnitt (1) ist auf einer Seite durch einen Spaltabschnitt (2) physikalisch vollständig für den Rauchstrom blockiert, wodurch es unmöglich ist, dass der durch die Verbrennung der organischen Substanz im Verbrennungsabschnitt (3) erzeugte Rauch in den Inhalationsabschnitt (1) gelangt, sodass dieses Inhalationsgerät keinen Rauchhauptstrom aufweist. Das bedeutet, dass der Benutzer bei der Verwendung des Inhalationsgeräts aufgrund des fehlenden Rauchhauptstroms keinen Rauch aus dem Inhalationsgerät einatmet. Damit der Inhalationsabschnitt (1) funktionieren kann, gelangt die äußere Umgebungsluft nur dann und nur durch perforierte Öffnungen in den Inhalationsabschnitt (1), wenn die organischen Stoffe im Verbrennungsabschnitt (3) für organische Stoffe verbrannt werden. Der Spaltabschnitt (2) besteht aus einer Dichtung, die an der Innenseite der Hülle des Spaltabschnitts angebracht werden kann, oder einer Dichtung, die an der Innenseite der Hülle des Spaltabschnitts befestigt ist, und der Spaltabschnitt (2) blockiert physisch vollständig den Rauchfluss auf einer Seite des Inhalationsabschnitts (1), so dass der durch die Verbrennung der organischen Substanz im Verbrennungsabschnitt (3) für organische Substanzen erzeugte Rauch nicht in den Inhalationsabschnitt (1) gelangt. Gleichzeitig blockiert der Spaltabschnitt (2) physisch vollständig den Rauchstrom auf einer Seite des Verbrennungsabschnitts (3) für organische Stoffe, um zu verhindern, dass der Hauptstrom des Rauchs in das Inhalationsgerät gelangt. Das bedeutet, dass der Rauch im Verbrennungsabschnitt (3) für organische Stoffe verbleibt und der Benutzer überhaupt keinen Rauch über das Inhalationsgerät einatmet. Der Verbrennungsabschnitt für organische Stoffe (3) wird auf einer Seite durch einen Spaltabschnitt (2) physisch vollständig für den Rauchfluss blockiert, sodass der durch die Verbrennung der organischen Stoffe entstehende Rauch nicht in den Inhalationsabschnitt (1) gelangen kann. Das bedeutet, dass das Inhalationsgerät keinen Rauchhauptstrom aufweist.

2. Ein Einweg-Inhalationsgerät, das Rauch freisetzt, der nicht direkt inhaliert wird, gemäß Anspruch 1, **dadurch gekennzeichnet, dass** der Spaltabschnitt (2) zwischen der inneren, für den Rauchfluss physikalisch blockierten Seite des Inhalationsabschnitts (1) und der inneren, für den Rauchfluss physikalisch blockierten Seite des Abschnitts (3) zur Verbrennung organischer Stoffe angeordnet ist.

3. Ein Einweg-Inhalationsgerät, das Rauch freisetzt, der nicht direkt inhaliert wird, gemäß den Ansprüchen 1 und 2, **dadurch gekennzeichnet, dass** der Spaltabschnitt (2) aus einer Dichtung besteht, die sich innerhalb der Hülle befindet. Die Dichtung besteht aus einem Materialtyp (13), der den Luft- oder Rauchfluss durch das Material verhindert, sodass die Bestandteile der Inhalationsvorrichtung, nämlich der Inhalationsabschnitt (1) und der Abschnitt zur Verbrennung organischer Stoffe (3), durch die Dichtung vollständig voneinander getrennt sind. Dadurch wird der Luft- oder Rauchfluss zwischen dem Inhalationsabschnitt (1) und dem Abschnitt zur Verbrennung organischer Stoffe (3) verhindert.

4. Einweg-Inhalationsgerät, das Rauch freisetzt, der nicht direkt inhaliert wird, gemäß den Ansprüchen 1, 2 und 3, **dadurch gekennzeichnet, dass** die Dichtung an der Innenseite (14) der Hülle angebracht ist und kein Luft- oder Rauchstrom in den Bereich zwischen der Dichtung und der Hülle zulässt, wodurch der Inhalationsbereich (1) und der Bereich zur Verbrennung organischer Stoffe (3) vollständig voneinander getrennt sind und kein Luft- oder Rauchstrom zwischen dem Inhalationsbereich (1) und dem Bereich zur Verbrennung organischer Stoffe (3) stattfindet.

5. Ein Einweg-Inhalationsgerät, das Rauch freisetzt, der nicht direkt inhaliert wird, gemäß den Ansprüchen 1 und 2, **dadurch gekennzeichnet, dass** der Spaltabschnitt (2) aus einer Dichtung besteht, die sich innerhalb der Hülle befindet und aus einem Materialtyp (15) besteht, der die Eigenschaft hat, weder Luft noch Rauch durchzulassen. Somit sind die Bestandteile des Inhalationsgeräts: Der Inhalationsabschnitt (1) und der Abschnitt zur Verbrennung organischer Stoffe (3) sind durch die Dichtung vollständig voneinander getrennt, sodass kein Luft- oder Rauchstrom zwischen dem Inhalationsabschnitt (1) und dem Abschnitt zur Verbrennung organischer Stoffe (3) stattfinden kann. Die Dichtung ist mit einem anderen Material an der Innenseite (16) der Hülle befestigt und verhindert, dass Luft oder Rauch in den Bereich zwischen der Dichtung und der Hülle strömen kann. Somit sind der Inhalationsabschnitt (1) und der Verbrennungsabschnitt für organische Stoffe (3) vollständig funktional voneinander getrennt, und es findet kein Luft- oder Rauchstrom zwischen dem Inhalationsabschnitt (1) und dem Verbrennungsabschnitt für organische Stoffe (3) statt.

6. Ein Einweg-Inhalationsgerät, das Rauch freisetzt, der nicht direkt inhaliert wird, gemäß den Ansprüchen 1 und 2, **dadurch gekennzeichnet, dass** der Trennteil (2) aus einer Dichtung besteht, die sich innerhalb der Hülle befindet und aus zwei oder mehreren verschiedenen Materialien (21) und (22) besteht, die die Eigenschaft haben, dass sie weder Luft noch Rauch durchlassen. Somit sind die Bestandteile des Inhalationsgeräts, nämlich der Inhalationsabschnitt (1) und der Abschnitt zur Verbrennung organischer Stoffe (3), durch die Dichtung vollständig voneinander getrennt, und es findet kein Luft- oder Rauchstrom zwischen dem Inhalationsabschnitt (1) und dem Abschnitt zur Verbrennung organischer Stoffe (3) statt.

7. Ein Einweg-Inhalationsgerät, das Rauch freisetzt, der nicht direkt inhaliert wird, gemäß den Ansprüchen 1, 2 und 6, **dadurch gekennzeichnet, dass** die Dichtung mittels eines anderen Materials an der Hülle befestigt oder an die Innenseite der Hülle geklebt ist und keinen Luft- oder Rauchstrom in dem Teil zwischen der Dichtung und der Hülle zulässt, wodurch der Inhalationsabschnitt (1) und der Verbrennungsabschnitt für organische Stoffe (3) vollständig funktional voneinander getrennt sind und kein Luft- oder Rauchstrom zwischen dem Inhalationsabschnitt (1) und dem Verbrennungsabschnitt für organische Stoffe (3) stattfindet.

8. Ein Einweg-Inhalationsgerät, das Rauch freisetzt, der nicht direkt inhaliert wird, gemäß den Ansprüchen 1 und 2, **dadurch gekennzeichnet, dass** der Spaltabschnitt (2) aus einer Dichtung besteht, die sich innerhalb der Hülle befindet und durch Versiegelung hergestellt wird. Der durch die zylindrische Hülle des Spaltabschnitts (2) gebildete Hohlraum wird mit einem Material versiegelt, das sich zum Zeitpunkt der Versiegelung in flüssigem oder annähernd flüssigem Zustand befindet und nach einer bestimmten Zeit aushärtet.

9. Einweg-Inhalationsvorrichtung, die Rauch freisetzt, der nicht direkt inhaliert wird, gemäß den Ansprüchen 1, 2 und 8, **gekennzeichnet durch** Folgendes: Nach dem Versiegelungsprozess hat das Material, sobald es ausgehärtet ist, die Eigenschaft, den Durchfluss von Luft oder Rauch zu verhindern. Somit sind der Inhalationsbereich (1) und der Bereich zur Verbrennung organischer Stoffe (3) durch die Versiegelung vollständig voneinander getrennt, sodass kein Luft- oder Rauchstrom zwischen dem Inhalationsbereich (1) und dem Bereich zur Verbrennung organischer Stoffe (3) möglich ist.

10. Ein Einweg-Inhalationsgerät, das Rauch freisetzt, der nicht direkt inhaliert wird, gemäß den Ansprüchen 1, 2, 8 und 9, **gekennzeichnet durch** Folgendes: Nachdem das Material, aus dem die Hülle besteht, ausgehärtet ist, haftet das Material an der Hülle des Spaltabschnitts (2) und verhindert so jeglichen Luft- oder Rauchstrom in dem Teil zwischen der Dichtung und der Hülle, wodurch der Inhalationsabschnitt (1) und der Verbrennungsabschnitt für organische Stoffe (3) vollständig funktional voneinander getrennt sind und kein Luft- oder Rauchstrom zwischen dem Inhalationsabschnitt (1) und dem Verbrennungsabschnitt für organische Stoffe (3) möglich ist.

11. Ein Einweg-Inhalationsgerät, das Rauch freisetzt, der nicht direkt inhaliert wird, gemäß Anspruch 1 und 2, **dadurch gekennzeichnet, dass** der Spaltabschnitt (2) aus einer Dichtung besteht, die sich innerhalb der Hülle befindet, und dass die Dichtung aus einem festen Material und Material (19) besteht und durch Abdichtung (18) hergestellt ist.

12. Einweg-Inhalationsgerät, das Rauch freisetzt, der nicht direkt inhaliert wird, gemäß den Ansprüchen 1, 2 und 11, **dadurch gekennzeichnet, dass** die Materialien (18) (19), aus denen die Dichtung besteht, die Eigenschaft haben, den Durchfluss von Luft oder Rauch zu verhindern, und somit der Inhalationsabschnitt (1) und der Verbrennungsabschnitt für organische Stoffe (3) durch die Dichtung vollständig funktional voneinander getrennt sind und keine Möglichkeit für einen Luft- oder Rauchstrom zwischen dem Inhalationsabschnitt (1) und dem Verbrennungsabschnitt für organische Stoffe (3) besteht.

13. Ein Einweg-Inhalationsgerät, das Rauch freisetzt, der nicht direkt inhaliert wird, gemäß den Ansprüchen 1, 2, 11 und 12, **dadurch gekennzeichnet, dass** die Dichtung mittels eines anderen Materials an der Hülle befestigt oder an die Innenseite der Hülle geklebt ist und keinen Luft- oder Rauchstrom in dem Teil zwischen der Dichtung und der Hülle zulässt, wodurch der Inhalationsabschnitt (1) und der Verbrennungsabschnitt für organische Stoffe (3) vollständig funktional voneinander getrennt sind und kein Luft- oder Rauchstrom zwischen dem Inhalationsabschnitt (1) und dem Verbrennungsabschnitt für organische Stoffe möglich ist (3) strömen kann.

14. Ein Einweg-Inhalationsgerät, das Rauch freisetzt, der nicht direkt inhaliert wird, gemäß Anspruch 1, 2, 3, 4, 5, 6, 7, 8, 9, 10, 11, 12 und 13, **dadurch gekennzeichnet, dass** das Material oder die Materialien, aus denen die Dichtung des Spaltabschnittes (2) besteht, teilweise oder vollständig in den Inhalationsabschnitt (1) und/oder den Verbrennungsabschnitt für organische Stoffe (3) eindringen.

## Revendications

1. Dispositif d'inhalation jetable libérant une fumée qui n'est pas directement inhalée, comprenant un corps d'inhalateur de forme cylindrique constitué d'une gaine en matériau mince non rigide ou en plusieurs types différents de matériaux minces non rigides, ledit corps cylindrique pouvant être déformé par l'application d'une pression externe, une extrémité du corps cylindrique étant remplie d'un matériau ou de plusieurs matériaux absorbant un liquide, lesdits matériaux étant non rigides et présentant la caractéristique de permettre l'écoulement de l'air à travers eux, dans lequel ou lesquels est disposée une capsule fragile, ladite capsule étant remplie d'une substance volatile d'inhalation ne nécessitant pas de chauffage pour s'évaporer, ou, alternativement, en l'absence de capsule cassable, le matériau ou les matériaux absorbant le liquide étant humidifiés ou imprégnés de gouttes d'une substance volatile d'inhalation ne nécessitant pas de chauffage pour s'évaporer, l'extrémité de la gaine où est disposé ledit matériau ou lesdits matériaux absorbant le liquide et permettant le passage de l'air étant perforée de manière à permettre, lors du processus d'inhalation, à l'air atmosphérique extérieur de pénétrer par des ouvertures perforées à l'intérieur de la partie contenant ledit matériau ou lesdits matériaux absorbant le liquide, l'autre extrémité du corps cylindrique étant remplie d'une matière organique combustible qui, lors de la combustion, brûle conjointement avec la gaine du dispositif d'inhalation dans la partie où est stockée ladite matière organique combustible,
**caractérisé en ce que** le dispositif d'inhalation comprend trois composants: la section d'inhalation (1), la section de séparation (2) et la section de combustion de matière organique (3), présentant les caractéristiques suivantes:
la section d'inhalation (1) est physiquement complètement bloquée pour le flux de fumée sur un côté par la section de séparation (2), empêchant la fumée produite par la combustion de la matière organique dans la section de combustion de matière organique (3) de pénétrer dans la section d'inhalation (1), de sorte que le dispositif d'inhalation ne présente aucun flux principal de fumée, l'utilisateur n'inhalant aucune fumée lors de l'utilisation du dispositif en raison de l'absence de flux principal de fumée;
afin que la section d'inhalation (1) fonctionne, l'air atmosphérique extérieur pénètre exclusivement par des ouvertures perforées lorsque la matière organique dans la section de combustion de matière organique (3) est en cours de combustion;
la section de séparation (2) comprend un joint pouvant être collé ou fixé à la face interne de la gaine de la section de séparation, ladite section de séparation (2) bloquant physiquement et complètement le flux de fumée du côté de la section d'inhalation (1) ainsi que du côté de la section de combustion de matière organique (3), empêchant l'apparition d'un flux principal de fumée dans le dispositif, la fumée demeurant dans la section de combustion de matière organique (3), l'utilisateur n'inhalant aucune fumée par le dispositif ;
la section de combustion de matière organique (3) est physiquement complètement bloquée pour le flux de fumée sur un côté par la section de séparation (2), empêchant toute pénétration de fumée dans la section d'inhalation (1), de sorte que le dispositif ne présente aucun flux principal de fumée.

2. Dispositif selon la revendication 1, **caractérisé en ce que** la section de séparation (2) est disposée entre le côté interne physiquement bloqué pour le flux de fumée de la section d'inhalation (1) et le côté interne physiquement bloqué pour le flux de fumée de la section de combustion de matière organique (3).

3. Dispositif selon les revendications 1 et 2, **caractérisé en ce que** la section de séparation (2) comprend un joint situé à l'intérieur de la gaine et constitué d'un seul type de matériau (13) présentant la caractéristique d'empêcher le passage de l'air ou de la fumée à travers celui-ci, de sorte que la section d'inhalation (1) et la section de combustion de matière organique (3) sont entièrement séparées fonctionnellement.

4. Dispositif selon les revendications 1 à 3, **caractérisé en ce que** le joint est fixé à la face interne (14) de la gaine et empêche tout passage d'air ou de fumée dans l'espace entre le joint et la gaine.

5. Dispositif selon les revendications 1 et 2, **caractérisé en ce que** la section de séparation (2) comprend un joint situé à l'intérieur de la gaine, constitué d'un matériau unique (15) empêchant le passage de l'air ou de la fumée, ledit joint étant fixé à la face interne (16) de la gaine par un autre matériau.

6. Dispositif selon les revendications 1 et 2, **caractérisé en ce que** la section de séparation (2) comprend un joint constitué de deux ou plusieurs matériaux différents (21, 22) empêchant le passage de l'air ou de la fumée.

7. Dispositif selon les revendications 1, 2 et 6, **caractérisé en ce que** le joint est fixé à la gaine au moyen d'un autre matériau ou collé à la face interne de la gaine, empêchant tout passage d'air ou de fumée entre le joint et la gaine.

8. Dispositif selon les revendications 1 et 2, **caractérisé en ce que** la section de séparation (2) est réalisée par scellement, la cavité formée par la gaine cylindrique étant remplie d'un matériau à l'état liquide ou quasi liquide au moment du scellement, lequel matériau durcit après un certain temps.

9. Dispositif selon les revendications 1, 2 et 8, **caractérisé en ce qu'**après durcissement, le matériau empêche le passage de l'air ou de la fumée.

10. Dispositif selon les revendications 1, 2, 8 et 9, **caractérisé en ce qu'**après durcissement, le matériau adhère à la gaine de la section de séparation (2), empêchant tout passage d'air ou de fumée entre le joint et la gaine.

11. Dispositif selon les revendications 1 et 2, **caractérisé en ce que** la section de séparation (2) comprend un joint constitué d'un matériau solide (19) et réalisé par scellement (18).

12. Dispositif selon les revendications 1, 2 et 11, **caractérisé en ce que** les matériaux (18, 19) empêchent le passage de l'air ou de la fumée.

13. Dispositif selon les revendications 1, 2, 11 et 12, **caractérisé en ce que** le joint est fixé à la gaine au moyen d'un autre matériau ou collé à la face interne de la gaine.

14. Dispositif selon les revendications 1, 2, 3, 4, 5, 6, 7, 8, 9, 10, 11, 12 et 13, **caractérisé en ce que** le joint est fixé à la gaine au moyen d'un autre matériau ou 13, **caractérisé en ce que** le ou les matériaux constituant le joint de la section de séparation (2) pénètrent partiellement ou totalement dans la section d'inhalation (1) et/ou dans la section de combustion de matière organique (3).
